(19) Europäisches Patentamt
European Patent Office
Office européen des brevets

(11) Veröffentlichungsnummer: **0 135 183**
**B1**

(12) # EUROPÄISCHE PATENTSCHRIFT

(45) Veröffentlichungstag der Patentschrift: **30.03.88**

(51) Int. Cl.⁴: **C 07 K 1/08**, C 07 K 5/00

(21) Anmeldenummer: **84110679.2**

(22) Anmeldetag: **07.09.84**

(54) **Verfahren zur Herstellung von Carbonsäureamidgruppen enthaltenden Verbindungen, insbesondere von Peptiden.**

(30) Priorität: **16.09.83 DE 3333456**

(43) Veröffentlichungstag der Anmeldung: **27.03.85 Patentblatt 85/13**

(45) Bekanntmachung des Hinweises auf die Patenterteilung: **30.03.88 Patentblatt 88/13**

(84) Benannte Vertragsstaaten: **AT BE CH DE FR GB IT LI LU NL SE**

(56) Entgegenhaltungen:
**EP - A - 0 056 618**
**DE - A - 2 901 843**

**ANGEWANDTE CHEMIE, Band 92, Seiten 129-130, Verlag Chemie, 1980, Weinheim; H. WISEMANN et al.: "Neue Peptidsynthese"**

(73) Patentinhaber: **HOECHST AKTIENGESELLSCHAFT, Postfach 80 03 20, D-6230 Frankfurt am Main 80 (DE)**

(72) Erfinder: **Wissmann, Hans, Dr., Falkenstrasse 12, D-6232 Bad Soden am Taunus (DE)**
Erfinder: **Teetz, Volker, Dr., An der Tann 20, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Zur Herstellung von Carbonsäureamid- und Peptidbindungen ist eine grosse Anzahl von Verfahren bekannt (siehe z.B. Houben-Weyl, Methoden der organischen Chemie, Bd. XV, Teil II, S. 1–364. Ferner Angew. Chemie 92, 129 (1980)) Alle diese Verfahren zielen mit unterschiedlichem Erfolg darauf, die für die Synthese von Peptiden notwendigen Kriterien der Racemisierungsfreiheit, der einfachen und schonenden Durchführbarkeit bei hohen Ausbeuten und mit leicht zugänglichen Ausgangsmaterialien sicherzustellen.

Aus der EP-A 156 618 ist ein Verfahren zur Herstellung von Carbonsäureamiden bekannt, bei welchem man Carboxylgruppen enthaltende Verbindungen mit Verbindungen, die eine freie Aminogruppe enthalten, in Gegenwart von Dialkylphosphinsäureanhydriden umsetzt. Die bei der Reaktion freigesetzte Dialkylphosphinsäure wird durch einen anfangs der Reaktionsmischung zugesetzten Überschuss einer organischen Base oder eines basischen Puffers gebunden.

Das vorliegende Verfahren stellt einen neuen Weg dar, die genannten Bedingungen für eine wirtschaftliche Synthese von Peptiden und Amiden zu optimieren.

Es wurde gefunden, dass man Carbonsäureamidgruppen enthaltende Verbindungen, insbesondere Oligopeptide, unter milden Bedingungen in guter Ausbeute herstellen kann, indem man Verbindungen, die eine freie Aminogruppe enthalten, insbesondere Aminocarbonsäurederivate oder Peptide, deren Carboxylgruppe geschützt ist, in Gegenwart des Anhydrids einer Dialkylphosphinsäure mit Verbindungen umsetzt, die eine freie Carboxylgruppe enthalten, insbesondere Aminocarbonsäuren oder Peptide, deren Aminogruppe acyliert ist. Das neue Verfahren ist dadurch gekennzeichnet, dass man die Wasserstoffionenkonzentration der Reaktionsmischung durch Zudosierung einer Base während der Reaktion bei einem annähernd konstanten Wert in einem Bereich von $10^{-5}$ bis $10^{-10}$ [mol/1] hält.

Die zum Schutz der funktionellen Gruppen eingeführten Reste können im Falle der Synthese von Peptiden anschliessend in üblicher Weise abgespalten werden.

Unter Anhydriden der Dialkylphosphinsäuren werden Verbindungen der Formel I verstanden

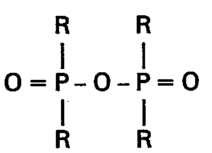

I,

in der R Alkyl bedeutet. Die in der Formel gezeigten Substituenten R können gleich oder verschieden sein. Bevorzugt sind Anhydride, bei denen beide P-Atome gleich substituiert sind.

Besonders geeignet im Sinne der Erfindung sind Anhydride der Formel I, in denen R jeweils ein niederes Alkyl ist, vorzugsweise ein solches mit 1 bis 4 C-Atomen.

Die erfindungsgemäss verwendeten Dialkylphosphinsäureanhydride sind farblose Flüssigkeiten. Sie sind bei Raumtemperatur stabil und lassen sich bei vermindertem Druck unzersetzt destillieren. In den meisten nicht wässrigen Lösungsmitteln, insbesondere in Lipidlösungsmitteln wie Chloroform oder Methylenchlorid, aber auch in polaren Lösungsmitteln wie DMF und DMA sind sie leicht löslich.

Als Anhydride der Dialkylphosphinsäuren seien beispielsweise genannt: Methyläthylphosphinsäureanhydrid, Methylpropylphosphinsäureanhydrid, Methyl-butylphosphinsäureanhydrid, Diäthylphosphinsäureanhydrid, Di-n-propylphosphinsäureanhydrid, Di-n-butylphosphinsäureanhydrid.

Die Herstellung der Dialkylphosphinsäureanhydride kann in an sich bekannter Weise erfolgen, z.B. durch Umsetzung der Dialkylphosphinsäurechloride mit Dialkylphosphinsäurealkylestern bei 150–160°C (Houben-Weyl, Methoden der Organischen Chemie, G. Thieme Verl., Stuttgart 1963, BD. XII, S. 266 ff). Besonders bevorzugt sind Verfahren, bei denen Dialkylphosphinsäuren, deren Salze oder Ester mit Phosgen umgesetzt werden (DBP 2 129 583, DOS 2 225 545).

Das erfindungsgemässe Verfahren wird vorzugsweise in gemischtwässrigen, ein- oder 2-phasigen Systemen innerhalb eines engen pH-Bereiches, bevorzugt bei annähernd konstantem pH-Wert, durchgeführt. Der pH-Wert der Reaktionsgemische kann bei 5–10, zweckmässigerweise soll er im Neutralen bis schwach Sauren liegen; insbesondere bevorzugt werden Werte zwischen 5,0 und 7,0. Jedoch sind auch Synthesen im schwach alkalischen Bereich möglich. Die Kontrolle des ph-Wertes erfolgt vorzugsweise durch dosierte Zugabe von konzentrierten wässrigen Lösungen der Alkalimetallhydroxyde, es können jedoch auch organische Basen wie N-Ethylmorpholin, Triethylamin oder Trialkylamine mit bis zu 6 C-Atomen verwendet werden.

Zur Herstellung von Oligopeptiden nach dem erfindungsgemässen Verfahren verwendet man als Ausgangsmaterialien einerseits eine Aminosäure oder ein Peptid mit einer blockierten Carboxylgruppe und andererseits eine Aminosäure oder ein Peptid mit einer blockierten Aminogruppe.

Für den Schutz der Carboxylgruppen können alle in der Peptidsynthese üblichen Schutzgruppen verwendet werden. Insbesondere eignen sich Ester geradkettiger oder verzweigter aliphatischer Alkohole wie Methanol, Äthanol, tert. Butanol. Auch Ester araliphatischer Alkohole wie Benzylalkohol oder Diphenylmethylcarbinol können Verwendung finden.

Zum Schutz der Aminogruppen können ebenfalls alle in der Peptidsynthese üblichen Schutzgruppen dienen. Als besonders geeignet seien beispielsweise der Carbobenzoxyrest und der Carbo-tert.-butyloxyrest genannt.

Als Lösungsmittel können alle in der Peptidsynthese üblichen wasserfreien, inerten Lösungsmittel, z.B. Methylenchlorid, Chloroform, Dimethylformamid, Dimethylacetamid, Dioxan oder Tetrahydrofuran Verwendung finden.

Als Lösungsmittel können bei der einphasigen, gemischtwässrigen Durchführung der Reaktion Gemische aus Wasser und einem mit Wasser mischbaren organischen Lösungsmittel, wie z.B. Dioxan, Tetrahydrofuran, Dimethylformamid oder Dimethylacetamid verwendet werden. Die Verwendung solcher Systeme ist insbesondere bei der Verknüpfung von überwiegend wasserlöslichen Peptiden von Vorteil.

Zur zweiphasigen, gemischtwässrigen Durchführung der Reaktion können Systeme wie z.B. Essigsäureethylester, Essigsäureisopropylester, Essigsäure-n-butylester, Methylenchlorid, Glykoldimethylether, 3-Methyl-tetrahydrofuran und Chloroform, jeweils in heterogener Mischung mit Wasser, verwendet werden.

Die Reaktion läuft in der Regel bei Raumtemperatur hinreichend schnell ab. Leichtes Erwärmen schadet nicht. Höhere Temperaturen, etwa oberhalb 30°C, sind, insbesondere bei der Peptidsynthese, wegen der Racemisierungsgefahr nicht zu empfehlen. Bevorzugt ist eine Reaktionstemperatur zwischen 0 und 30°C, insbesondere eine solche zwischen 5 und 25°C.

Das erfindungsgemässe Verfahren erlaubt es erstmalig, den Verlauf der Umsetzung anhand des Verbrauchs der eingesetzten Base bei annähernd konstantem pH-Wert zu verfolgen. Dazu wird die Reaktion vorzugsweise in einem selbstschreibenden pH-Staten zweckmässigerweise unter kräftiger Durchmischung des die Reaktionspartner Carboxylkomponente, Aminkomponente und Dialkylphosphinsäureanhydrid enthaltenden gemischtwässrigen Systems durchgeführt. Die vom Schreiber gelieferte Darstellung des Basenverbrauches in Abhängigkeit von der Zeit zeigt eine Kurve, die gegen Ende der Reaktion asymptotisch verläuft (vergl. die Kurvenreproduktion in der Fig.).

Das erfindungsgemässe Verfahren bietet also die Möglichkeit, den Endpunkt der Synthesereaktion auf einfache Weise zu erkennen.

Es sei angemerkt, dass die nach der beschriebenen Methode in diesen gemischtwässrigen Systemen gemessenen «scheinbaren» pH-Werte von den echten pH-Werten dieser Systeme abweichen können.

Verwendet man in bekannter Weise Pufferlösungen zum Abfangen der Dialkylphosphinsäure, so treten zwangsweise aus anorganischen Salzen zusammengesetzte Salzüberschüsse in den Mutterlaugen der Ansätze auf. Sie erschweren bei der technischen Durchführung des Verfahrens die Rückgewinnung der aus dem Kondensationsmittel entstandenen Dialkylphosphinsäure, und können zu Abwasserproblemen Anlass geben.

Die bei der Verwendung von Alkalihydroxyden als Basen bei dieser Variante des erfindungsgemässen Verfahren im Verlauf der Reaktion gebildeten Alkalisalze sind im Gegensatz zu den bei der Verwendung mancher organischer tertiärer Basen entstehenden Salze bei einer Isolierung des Reaktionsproduktes durch Extraktion aus alkalischer Lösung mit einer mit Wasser nur beschränkt mischbaren Lipoidphase infolge ihrer grösseren Hydrophilität leichter abzutrennen. Ferner wird bei dieser Verfahrensweise eine Verunreinigung der Lipidphase durch extrahierte tertiäre Base vermieden.

Das erfindungsgemässe Verfahren ist einfach durchzuführen und liefert Peptide in hoher optischer Reinheit und Ausbeute. Ausserdem ist es sparsam und umweltfreundlich.

Die Dialkylphosphinsäureanhydride sind niedrigmolekular, leicht zu erhalten und zu reinigen und zeigen einen hohen Anteil an reaktiven Gruppen pro Gewichtseinheit sowie eine gute Lipophilität. Die Dialkylphosphinsäureanhydride und die zugehörigen Dialkylphosphinsäuren sind lipidlöslich. Das ermöglicht eine Aufarbeitung wasserlöslicher Peptidderivate über einen ersten Fällungsschritt mit geeigneten Lipoidlösungsmitteln.

Die aus dem Dialkylphosphinsäureanhydrid im Verlauf der Peptidsynthese erhaltene Dialkylphosphinsäure kann aus den Restlösungen der Synthesereaktion wiedergewonnen werden. Eine Wiedergewinnung der Dialkylphosphinsäuren aus einer grösseren Menge von wässrigen Synthesemutterlaugen ist durch Extraktion mit Lösungsmitteln wie Chloroform und Isobutanol und anschliessender destillativer Aufarbeitung möglich. Dabei ist es von besonderem technischen Interesse, dass die Dialkylphosphinsäuren im Vakuum ohne Zersetzung destillierbar sind. Die so gewonnenen wiederaufgearbeiteten Dialkylphosphinsäuren können sodann leicht nach dem Verfahren der DOS 2 225 545 in die entsprechenden Dialkylphosphinsäureanhydride übergeführt werden.

Bei der beschriebenen gemischtwässrigen Arbeitsweise kann die organische Base durch Alkalihydroxydlösungen ersetzt werden, was die oben beschriebene Wiederaufarbeitung der Dialkylphospinsäuren nach der Synthese wesentlich vereinfacht. In diesem Fall kann auf den Extraktionsschritt verzichtet werden. Die Dialkylphosphinsäure kann dann nach Freisetzung direkt aus der abgedampften Synthesemutterlauge abdestilliert werden, oder wird bei zweiphasiger Arbeitsweise nach dem Abtrennen der Lipoidphase durch Ansäuern und Extraktion aus der wässrigen Phase zurückgewonnen. In der wässrigen Phase verbleiben dann die anorganischen Neutralsalze.

Beispiel 1

Carbobenzoxy-glyzinethylester:

Zu der Lösung von 10,5 g (0,05 Mol) Carbobenzoxyglyzin in 120 ml Essigsäureethylester gibt man unter Rühren bei -5°C 7,0 g (0,05 Mol) H-Gly-$OCH_3 \cdot HCl$. Zu der entstandenen Suspension tropft man 20 g Methylethylphosphinsäureanhydrid. Bei einer Temperatur von 0' bis +10°C stellt man dann den pH-Wert der Reaktionsmischung mit Hilfe eines Autotitrators durch Zutropfen von 4n NaOH auf 7,0, und hält den durch hochtouriges Rühren gut durchgemischten Ansatz bei diesem ph, bis der an den Autotitrator angeschlossene Schreiber einen asymptotischen Verlauf der Natronlauge-Verbrauchskurve gegen die Zeitachse zeigt. Das ist nach etwa 60 Minuten der Fall. Man trennt nun die Essigesterphase ab, extrahiert sie mit zweimal 50 ml gesättigter Natriumbicarbonatlösung, trocknet mit Natriumsulfat, und erhält nach Abdampfen des Lösungsmittels im Vakuum bei Raumtemperatur 12,25 g (84% d. Th.) Z-Dipeptidester mit einem Schmelzpunkt von 81°C.

Beispiel 2

Carbobenzoxy-phenylalanin-cyclohexylamid:

Man löst 3,0 g (0,01 Mol) Z-Phe-OH und 1,0 g (0,01 Mol, 1,2 ml) Cyclohexylamin in einem Gemisch aus 20 ml Tetrahydrofuran und 5 ml Wasser. Nach Abkühlen des Reaktionsgemisches auf -5°C setzt man unter Rühren 4 g Memthylethylphosphinsäureanhydrid zu, stellt den pH der Reaktionslösung mit 4n NaOH auf 6,0 und hält es durch dosierte Natronlaugezugabe wie in Beispiel 1 beschrieben, während der gesamten Umsetzungszeit konstant. Nach 60 Minuten ist die Reaktion, wie aus dem Alkalihydroxydverbrauch ersichtlich, praktisch beendet. Man engt die Reaktionslösung im Vakuum bei Raumtemperatur ein, nimmt mit Essigester auf, und wäscht die Essigesterlösung mit 5%iger Kaliumbisulfatlösung, gesättigter Natriumbicarbonatlösung und Wasser, trocknet über Natriumsulfat und erhält nach Abdampfen des Lösungsmittels im Vakuum bei Raumtemperatur und nach Trocknen des Produktes im Vakuum über $P_2O_5$ 3,0 g Endprodukt vom Fp 167°C, $[\alpha]_D = -3{,}0°$ (c=1, DMF).

Beispiel 3

Z-Trp-Gly-$OCH_3$

Man löst 3,35 g (0,01 Mol) Z-Trp-OH in 20 ml Essigsäureisopropylester, gibt 1,25 g (0,01 Mol) H-Gly-$OCH_3$ zu, kühlt die gut gerührte Suspension auf -5°C und gibt bei dieser Temperatur unter gleichzeitiger Einstellung des pH-Wertes auf 5,7 durch dosierte Zugabe von 4n NaOH am Autotitrator wie in Beispiel 1 beschrieben, 4,0 ml Methylethylphosphinsäureanhydrid zu. Die Reaktion wird unter guter Durchmischung der Phasen innerhalb von 60 Minuten zu Ende geführt. Die abgetrennte Essigesterphase wird wie in Beispiel 2 beschrieben, aufgearbeitet.

Ausbeute: 3,53 g (86% d. Th.) $[\alpha]_D$-13,5° (c=0,1 Eisessig).

Beispiel 4

Z-Phe-Arg-Trp-Gly-$OCH_3$

Zu der Lösung von 2,26 g (0,005 Mol) Z-Phe-Arg-OH in 25 ml Eisessigsäureisopropylester gibt man 1,55 g (0,005 Mol) H-Trp-Gly-$OCH_3$·HCl und kühlt die gut gerührte Suspension auf -5°C. Dann tropft man 2 ml Methylethylphosphinsäureanhydrid unter Konstanthalten des pH-Wertes bei pH 5,2 mit 4n NaOH wie im Beispiel 1 beschrieben, zu. Nach 15 Minuten lässt man die Temperatur des Reaktionsgemisches auf Raumtemperatur kommen. Nach 70 Minuten ist die Umsetzung praktisch vollständig, wie die Aufzeichnung des NaOH-Verbrauches gegen die Reaktionszeit zeigt. Man trennt nun die Essigesterphase ab, wäscht sie mit Wasser und gesättigter Natriumbicarbonatlösung und isoliert das Reaktionsprodukt aus der getrockneten Lösung durch Abdampfen i.V. bei Raumtemperatur und Digerieren des Rückstandes mit abs. Diethylether.

Ausbeute nach Umkristallisieren aus Ethanol/Ether: 3,0 g $[\alpha]_D = -25{,}7°$ (c=0,1, DMF). (84,5% d.Th.)

Beispiel 5

Z-Lys(Boc)-Val-Tyr-$OCH_3$

Man löst 1,91 g (0,005 Mol) Z-Lys(Boc)-OH in 25 ml mit Wasser gesättigtem Butylacetat, gibt 1,65 g (0,005 Mol) H-Val-Tyr-$OCH_3$·HCl zu, kühlt die gut gerührte Reaktionsmischung auf -5°C, und bringt dann den pH-Wert der schnell gerührten Mischung mit 4n NaOH wie im Beispiel 1 beschrieben, auf 7,0, und hält es während der ganzen Reaktionsdauer (70 Minuten) auf diesem Wert. Die Temperatur hält man während der ersten 10 Minuten der Reaktionszeit auf -5°C bis 0°C, dann lässt man auf Raumtemperatur erwärmen. Die Aufarbeitung der das Endprodukt enthaltenden Butylacetatphase erfolgt wie im Beispiel 2 angegeben.

Ausbeute nach Umkristallisation aus Ethanol/Ether: 3,0 g (91% d. Th.)

$[\alpha]_D = -25°$ (c=1, Ethanol).

Beispiel 6

Z-Gly-Leu-Arg-$OCH_3$

Die Lösung von 642 mg Z-Gly-Leu-OH und 449 mg H-Arg-OMe. HCl in 3 ml Dimethylacetamid und 0,5 ml Wasser versetzt man mit 1,3 ml Methylethylphosphinsäureanhydrid und hält den pH-Wert während der nun ablaufenden Reaktion mit einer Mischung aus N-Ethylmorpholin und Wasser (1:1, Vol/Vol) bei 7,2 durch einen pH-Staten. Nach 40 Minuten ist lt. Schreiber-Aufzeichnung die Reaktion beendet. Die Aufarbeitung erfolgt wie im Beispiel 2 beschrieben, jedoch ohne die dort angeführte Extraktion der Essigesterphase mit 5% Kaliumbisulfatlösung.

Ausbeute: 700 mg (71% d. Th.)

$[\alpha]_D = -24{,}4°$ (c=1, $CH_3OH$)

**Patentansprüche**

1. Verfahren zur Herstellung von Carbonsäureamidgruppen enthaltenden Verbindungen durch Umsetzung von Verbindungen, die eine Carboxylgruppe enthalten, in Gegenwart von Dialkylphosphinsäureanhydriden mit Verbindungen, die eine freie Aminogruppe enthalten, dadurch gekennzeichnet, dass man die Wasserstoffionenkonzentration der Reaktionsmischung durch Zudosierung einer Base während der Reaktion bei einem annähernd konstanten Wert in einem Bereich von $10^{-5}$ bis $10^{-10}$ [mol/l] hält und nach beendeter Reaktion gegebenenfalls zum

Schutz anderer funktioneller Gruppen eingeführte Reste abspaltet.

2. Verfahren nach Anspruch 1 zur Herstellung von Peptiden.

3. Verfahren nach Anspruch 1 oder 2, dadurch gekennzeichnet, dass die Reaktion in einem homogenen oder heterogenen gemischtwässrigen Medium durchgeführt wird.

4. Verfahren nach einem der Ansprüche 1 bis 3, dadurch gekennzeichnet, dass als Basen wässrige Lösungen der Alkalimetallhydroxide verwendet werden.

5. Verfahren nach einem der Ansprüche 1 bis 3, dadurch gekennzeichnet, dass als Basen Trialkylamine verwendet werden.

6. Verfahren nach einem der Ansprüche 1 bis 5, dadurch gekennzeichnet, dass der Endpunkt der Synthesereaktion mit Hilfe der Schreiberaufzeichnung eines pH-States (Basenverbrauch gegen die Reaktionszeit) festgestellt wird.

7. Verfahren nach einem der Ansprüche 1 bis 6, dadurch gekennzeichnet, dass die Reaktion zwischen 0 und 30°C durchgeführt wird.

**Claims**

1. A process for the preparation of compounds containing carboxamide groups by reaction of compounds which contain a carboxyl group, in the presence of dialkylphosphinic anhydrides, with compounds which contain a free amino group, which comprises maintaining the hydrogen ion concentration in the reaction mixture at an almost constant value in a range of $10^{-5}$ to $10^{-10}$ (mol/l) during the reaction and, after the reaction is complete, eliminating radicals which have, where appropriate, been introduced to protect other functional groups.

2. The process as claimed in claim 1 for the preparation of peptides.

3. The process as claimed in claims 1 or 2, wherein the reaction is carried out in a homogeneous or heterogenous mixed aqueous medium.

4. The process as claimed in one of claims 1-3, wherein the bases used are aqueous solutions of alkali metal hydroxides.

5. The process as claimed in one of claims 1-3, wherein trialkylamines are used in the bases.

6. The process as claimed in one of claims 1-5, wherein the end-point of the synthetic reaction is established with the aid of the graph recorded by a pH-stat (consumption of base versus reaction time).

7. The process as claimed in one of claims 1-6, wherein the reaction is carried out between 0-30°C.

**Revendications**

1. Procédé pour la préparation de composés contenant des groupes amide d'acide carboxylique, par réaction de composés qui contiennent un groupe carboxy, avec des composés qui contiennent un groupe amino libre, en présence d'anhydrides dialkylphosphiniques, caractérisé en ce que l'on maintient pendant la réaction la concentration des ions hydrogène du mélange réactionnel à une valeur pratiquement constante dans une plage de $10^{-5}$ à $10^{-10}$ (mole/litre) par addition réglée d'une base, et une fois la réaction terminée, on élimine éventuellement des radicaux introduits pour la protection d'autres groupes fonctionnels.

2. Procédé selon la revendication 1, pour la préparation de peptides.

3. Procédé selon la revendication 1 ou 2, caractérisé en ce que l'on effectue la réaction dans un milieu aqueux mixte homogène ou hétérogène.

4. Procédé selon l'une des revendications 1 à 3, caractérisé en ce que l'on utilise, en tant que bases, des solutions aqueuses des hydroxydes de métaux alcalins.

5. Procédé selon l'une des revendications 1 à 3, caractérisé en ce que l'on utilise, en tant que bases, des trialkylamines.

6. Procédé selon l'une des revendications 1 à 5, caractérisé en ce que l'on détermine le point final de la réaction de synthèse à l'aide de l'enregistrement graphique d'un pH-stat (consommation de la base en fonction du temps de réaction).

7. Procédé selon l'une des revendications 1 à 6, caractérisé en ce que l'on effectue la réaction entre 0 et 30°C.

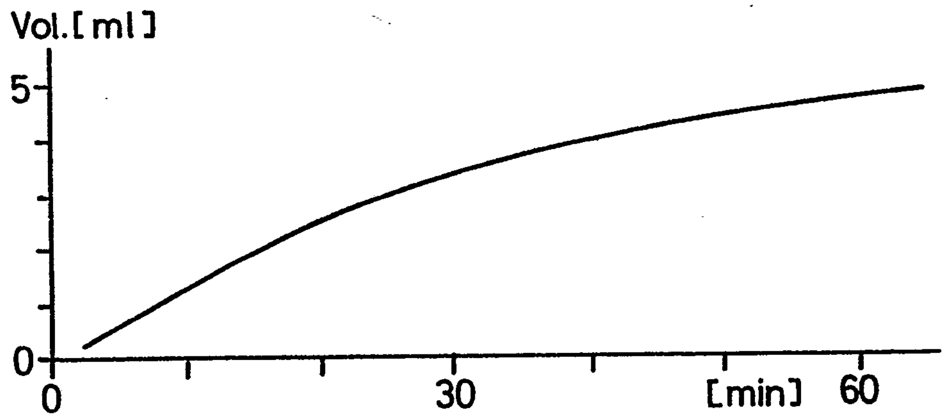
Vol.[ ml ]
5
0
0
30
[min]
60